# FASCICULE DE BREVET EUROPEEN

(11) **EP 0 302 801 B1**
(45) Date de publication et mention de la délivrance du brevet: **18.10.1995**
(21) Numéro de dépôt: 88402057.9
(22) Date de dépôt: 05.08.1988
(51) Int. Cl.: C12N 15/63, C12N 15/86, C12N 7/00, C07H 21/00, A61K 39/12

(54) **Vaccins dont l'épitope caractéristique est incorporé dans une protéine de picornavirus, notamment de poliovirus**
Impfstoffe, deren charakteristischer Epitop in einem Picornavirusprotein, insbesondere Poliovirus, inkorporiert ist
Vaccines of which the characteristic epitope is incorporated in a picorna virus protein, particularly in that of the polio virus

(30) Priorité: 07.08.1987 FR 8711337
(43) Date de publication de la demande: 08.02.1989
(73) Titulaire: INSTITUT PASTEUR, 75724 Paris Cédex 15 (FR)
(72) Inventeur: Girard, Marc, F-75015 Paris, (FR); Wychowski, Czeslaw,, F-62410 Meurchin, (FR); Martin, Annette,, F-75015 Paris, (FR)
(74) Mandataire: Gutmann, Ernest

(56) Documents cités:
- WO-A-83/02393
- J. Virol. (1985), p.786-792, Kohara et al.
- J. Virol. (1986), p.1187-1190, Stanway et al.

## Description

Le poliovirus, connu comme étant l'agent d'une maladie infectieuse humaine, la poliomyélite ou maladie de Heine-Madin, a été étudié comme prototype des virus de la famille des picornaviridae. Depuis quelques années, l'essor du génie génétique a contribué à une meilleure connaissance de ce virus et a permis le clonage moléculaire de l'ARN viral en cADN et la détermination nucléotidique de son génome (Kitamura et al.1981 ; Racaniello et Baltimore, 1981, van der Werf et al., 1981). En outre, la possibilité d'induire un cycle infectieux dans les cellules de primates, à partir de plasmide porteur d'une copie complète du génome viral, a contribué à l'élaboration d'autres vecteurs dont l'infectivité spécifique a pu être augmentée (Semler et al., 1984, Kean et al., 1986). L'étude des autres picornavirus progresse rapidement aussi. On rappellera qu'à cette classe de virus se rattachent les entérovirus, dont les poliovirus de types 1, 2 et 3 constituent une sous-classe. Ils englobent aussi les cocksackievirus et les echovirus, les rhinovirus, les cardiovirus, les aphtovirus et le virus de l'hépatite virale A. Les génomes des picornavirus sont des ARNs dont la taille est en général de l'ordre de 7500 nucléotides.

Les travaux de Hogle et al., (1985) joints à ceux de Rossmann (1985) montrent qu'il existe trois sites majeurs de neutralisation chez les picornavirus : NImI sur VP1, NImII sur VP2 et VP1 et NImIII sur VP3 et VP2 (NIm étant une abréviation de l'expression anglaise "neutralization antigen", donc en français : "antigène de neutralisation"). Chez le poliovirus de type 1, on a mis en évidence un site NImI (acide aminés 93-104, Wychowski et al., 1983) dont l'épitope C3 est séquentiel, et des sites NImII et NImIII qui déterminent des sites conformationnels à la surface du virion.

Pour ce qui est de la localisation et de l'identification d'autres épitopes immunogènes, on peut encore, pour les poliovirus, se reporter aux travaux de E.A.Emini, A.Jameson, A.J.Lewis, G.R.Larsen, E.A.Wimmer, J.Virol.43.997(1982); R.Crainic et al., Infect.Immun. 41.1217(1983); P.Minor et al., Nature (London) 301.674 (1983); P. Minor et al. J.Gen. Virol. 65.1159 (1985); M.Fergusson et al., Virology 143.505 (1985); D.C.Diamond et al.Science 229.1090 (1985); D.M.A. Evans et al., Nature (London) vol. 304,459-462.

De même, on peut se reporter, par exemple, aux articles de Rossmann M.A. et al., (1985) ;Sherry B et al., J. Virol 57, 246-257 (1986)DUECHLER et al.Proc.Natl. Acad.Sci. USA, vol. 84, pp.2605-2609 (1987) pour ce qui est des études menées pour localiser et identifier des épitopes neutralisants portés par les rhinovirus.

Si la plupart des épitopes immunogènes identifiés à ce jour sont portés par les capsides des virus concernés, à la surface de ces capsides, il en est d'autres qui se sont révélés comme susceptibles d'induire des réponses neutralisantes significatives, par exemple chez le rat et le lapin sans être à la surface. Le peptide correspondant à la séquence de résidus d'aminoacides 113-121 de VP1, dont une partie seulement est exposée (B.A. Jameson et al, dans l'ouvrage intitulé "vaccines" (vaccins) de R.Lerner et al, Eds. (Cold Spring Harbor Laboratory, Cold Spring Harbopr N.Y. 1985) pp. 191-198), ou des peptides internes aux virus, par exemple ceux correspondant aux séquences de résidus d'aminoacides 61-80 et 180-201 du poliovirus ont ainsi induit des anticorps aux propriétés neutralisantes importantes.

L'invention repose sur l'idée d'utiliser un picornavirus susceptible de se répliquer dans les cellules de l'hôte à vacciner contre un agent pathogène déterminé, comme vecteur porteur d'un épitope, hétérologue vis-à-vis de ce picornavirus, immunogène susceptible d'induire chez l'hôte la production d'anticorps neutralisants contre cet agent pathogène, ledit épitope étant incorporé à une protéine du picornavirus, en lieu et place d'un épitope immunogène, neutralisant à l'égard de ce picornavirus.

Le picornavirus utilisable en tant que vecteur est le poliovirus.

En d'autres termes l'invention concerne par conséquent un poliovirus hybride viable contenant, en lieu et place de l'epitope situé entre les acides aminés 93 et 104 de la protéine VP1 ou d'une partie de cet épitope, un épitope caractéristique d'une protéine vaccinante hétérologue vis-à-vis des protéines de ce poliovirus, tous les autres épitopes normalement exposés du poliovirus étant reconnus par les anticorps spécifiques qui leur correspondent et produits à partir du poliovirus natif ou de protéines obtenues à partir de celui-ci.

D'une façon générale il peut être dit aussi que l'épitope hétérologue se trouve incorporé par construction dans la protéine porteuse qui initialement contenait l'épitope endogène, immunogène de ce poliovirus. De préférence on l'a vu, l'épitope hétérologue est exposé à la surface de la capside.

Dans un mode préféré de mise en oeuvre de l'invention, le picornavirus vecteur de la capside du poliovirus est un poliovirus du type 1 dans lequel l'épitope hétérologue est substitué à l'épitope C3 normalement exposé à la surface de la capside du poliovirus.

L'invention concerne également les produits intermédiaires intervenant dans la construction des poliovirus hybrides viables, plus particulièrement les cADNs hybrides recombinants comprenant la totalité ou tout au moins les parties de cADNs de poliovirus nécessaires à la production d'un virus viable dans une culture de cellules compétentes dans lesquelles le poliovirus non transformé est cultivable, d'une part, et une séquence de nucléotides hétérologue, le cas échéant synthétique, codant pour un épitope distinct, substituée dans ce cADN à une séquence d'ADN endogène ou naturelle codant pour une séquence d'acides aminés immunogène, d'autre part. La séquence hétérologue est substituée à une séquence endogène qui, dans le poliovirus non modifié et compte tenu de la nature des séquences environnantes, codait pour l'épitope situé entre les acides aminés 93 et 104 de la protéine VP1 ou d'une partie de cet epitope.

Elle concerne plus particulièrement des plasmides recombinants dans lesquels les cADNs hybrides susmentionnés ont été incorporés par construction, sous le contrôle d'un promoteur et d'éléments de régulation autorisant la replication autonome des poliovirus recombinants correspondants dans des organismes procaryotes ou eucaryotes utilisables pour la production de quantités importantes de poliovirus.

A ce titre, l'invention concerne plus particulièrement des plasmides porteurs de cADNs recombinants dérivés des cADNs de poliovirus, modifiés dans les conditions précédemment indiquées, par un polynucléotide codant pour un épitope immunogène hétérologue, en particulier des plasmides utilisables pour la transformation d'organismes cellulaires procaryotes ou eucaryotes et permettant la réplication autonome et la production du poliovirus, modifié correspondant.

L'invention concerne aussi des ARNs recombinants contenant la totalité de l'ARN messager du poliovirus correspondant y incluses les séquences codant pour les protéines VP0, VP3 et VP1, et comprenant la substitution correspondante à celle du cADN correspondant. Cet ARN messager peut être obtenu soit in vitro, soit en cultures de cellules préalablement transformées, à partir d'un vecteur contenant le cADN du poliovirus correspondant, sous le contrôle d'un promoteur et d'éléments de régulation appropriés.

Il est tout à fait remarquable que les modifications ainsi introduites dans la protéine VP1 des poliovirus, peuvent être réalisées, dans la plupart des cas, sans nuire à leur viabilité. L'invention fournit par conséquent des principes actifs utilisables pour la constitution de "vaccins vivants" ou inactivés à partir d'épitopes caractéristiques codés par des oligonucléotides qui peuvent avoir été produits par synthèse. Ces vaccins peuvent naturellement aussi être produits à partir d'une séquence naturelle de nucléotides, obtenue par clivage à partir de l'ADN naturel ou d'un cADN dérivé de l'ARN naturel codant pour la protéine qui contient normalement la séquence d'aminoacides de l'épitope.

L'invention concerne également les vaccins euxmêmes obtenus à partir des virus sus-indiqués notamment par inactivation de leur virulence par des méthodes classiques ou des vaccins vivants de virulence atténuée.

On observera que les méthodes applicables à l'inactivation du poliovirus pour la constitution de vaccins contre la poliomyélite sont applicables de la même manière; on rappellera pour mémoire et à titre d'exemple seulement, les traitements thermiques par exemple entre 50 et 60°C, les traitements par des agents chimiques tels que formol ou autres aldéhydes appropriés qui peuvent être utilisés pour obtenir ces vaccins inactivés.

L'intérêt majeur du vaccin selon l'invention réside dans la possibilité de mettre en jeu les caractéristiques parfaitement maîtrisées ou maîtrisables des poliovirus, et des vaccins contre ces picornavirus, notamment des vaccins contre la poliomyélite pour la fabrication d'autres vaccins. En outre, les épitopes hétérologues des nouveaux vaccins se trouvent présentés à l'organisme de l'hôte vacciné sous une structure tridimensionnelle apparentée à la forme immunogène naturelle des poliovirus.

L'invention concerne enfin un procédé pour la production des poliovirus hybrides sus-définis, ce procédé étant caractérisé en ce que, partant d'un cADN obtenu à partir de l'ARN viral,
- on introduit de part et d'autre de la séquence codant pour l'épitope situé entre les acides aminés 93 et 104 de la protéine VP1 d'un poliovirus ou pour une partie de cet épitope, des sites de restriction spécifiques dans le cADN ;
- on clive le cADN au niveau de ces sites spécifiques par les enzymes de restriction correspondantes ;
- on reconstitue un cADN recombinant hybride formé, d'une part, à partir de tous les éléments de l'ARN viral ou du cADN nécessaire à la réplication ultérieure du virus et, d'autre part, une séquence codant pour un épitope immunogène de substitution en lieu et place de la séquence codant pour l'épitope naturel ;
- on transfecte une culture cellulaire sensible avec cet ADN recombinant ;
- et l'on recueille enfin le poliovirus recombinant correspondant.

Pour ce qui est de l'information des sites spécifiques entourant la séquence codant pour l'épitope immunogène endogène à substituer, on peut procéder par toutes méthodes appropriées visant à introduire un site de restriction spécifique à un endroit déterminé d'un acide nucléique. Avantageusement, l'on procèdera par mutagenèse dirigée, par exemple comme décrit dans l'exemple ci-après.

Afin de s'assurer du clivage spécifique aux endroits voulus de l'ADN à traiter, et ce en l'absence de clivages parasites dans d'autres régions de cet ADN, celui-ci aura de préférence été modifié au préalable en ces autres endroits pour faire disparaître les sites de restrictions semblables qui pouvaient y être présents. Cette dernière opération peut être réalisée par toute méthode appropriée permettant la modification des sites de restriction ou encore par mutagénèse dirigée.

Dans ce qui précède, l'on n'a évoqué qu'une substitution limitée du cADN du poliovirus correspondant. L'homme du métier appréciera que l'on peut encore envisager la production de virus hybrides plus profondément transformés pour répondre aux besoins de la pratique. Les techniques sus-indiquées permettent par exemple le greffage d'une partie de la séquence complète codant pour les protéines successives, VP0, VP3 et VP1, d'un rhinovirus ou du HAV dans le cADN d'un poliovirus, en lieu et place des séquence correspondantes codant pour l'épitope situé entre les acides aminés 93 et 104 de la protéine VP1 du poliovirus.

Il est également nécessaire dans ce cas-là de modifier les sites spécifiques de coupure dans le cADN du rhinovirus ou du HAV par mutagenèse dirigée, afin que les clivages nécessaires à la production ultérieure du virus hybride obtenu entre les protéines VP0 et VP3 d'une part, et entre les protéines VP3 et VP1 d'autre part, puissent être effectués sous le contrôle de la séquence codant la protéase spécifique qui, dans le poliovirus naturel, coupe les liaisons glutamine-glycine séparant les protéines sus-indiquées.

Cette transformation permet en conséquence l'obtention d'un picornavirus dérivé du poliovirus, mais pourvu d'une partie de la capside de rhinovirus, ou du HAV.

Il apparaîtra clairement à l'Homme du Métier que des constructions faisant intervenir d'autres associations de différents picornavirus peuvent être envisagées de la même manière, au gré du constructeur, et de la compatibilité maximum recherchée entre le virus hybride produit et l'organisme de l'hôte à vacciner.

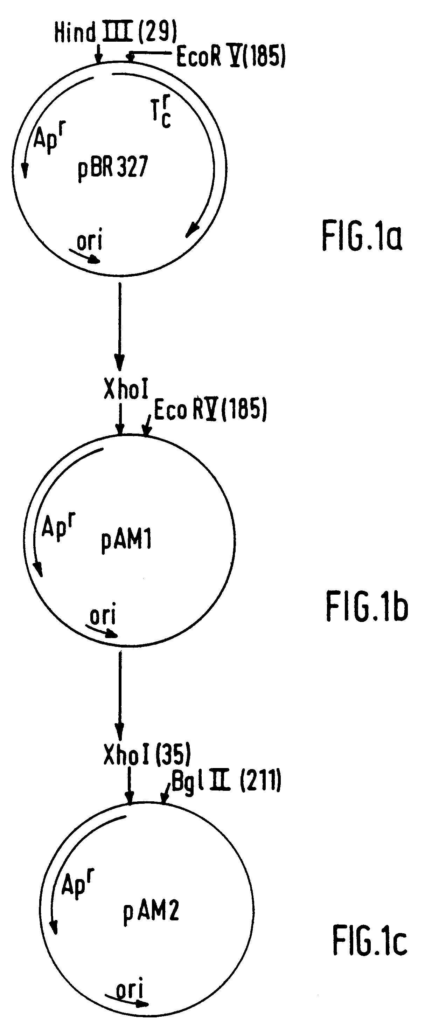

Des caractéristiques supplémentaires de l'invention apparaîtront encore au cours de la description qui suit, de mode de construction d'un ADN recombinant hybride conforme à l'invention dérivé d'un poliovirus de type 1, en s'appuyant sur les figures 1a à 5d qui illustrent schématiquement les étapes successives de cette construction et les structures des différents plasmides mis en oeuvre.

### I CONSTRUCTION DU VECTEUR DE SOUS CLONAGE pBR327 MODIFIE

Le plasmide pBR327 (SOBERON et al. 1980) (fig 1a) capable de se répliquer dans les bactéries E. coli, portant les gènes de résistance à l'ampicilline (Ap^{r}) et à la tétracycline(Tc^{r}) et son origine de réplication (ori), est choisi comme vecteur pour effectuer les constructions. Ce vecteur est modifié pour les besoins du clonage de la façon suivante.

### 1° Remplacement du site HindIII (position 29) de ce plasmide par un site XhoI.

Le vecteur est ouvert au site HindIII par l'enzyme de restriction correspondante (BOEHRINGER, conditions d'utilisation fournies par la firme), les bouts cohésifs générés (40 µg DNA/ml) sont remplis par traitement à la DNA polymérase de KLENOV (100 U/ml) en présence des 4 nucléotides (dNTP) (200 µM chacun) selon le protocole décrit par MANIATIS et al. (1982). L'ADN linéarisé est déphosphorylé par traitement à la phosphatase alcaline d'intestin de veau (2 x 0,02 unités par µg de DNA) dans un tampon 50 mM Tris-HCl pH 8,0 10 mM MgCl₂, 0,1 mM EDTA, 1 mM ZnCl₂ (enzyme et protocole d'utilisation BOEHRINGER) deux fois pendant 30 minutes à 37°C, avant ligation avec un adapteur (linker) synthétique Xhol phosphorylé (8 nucléotides, commercialisé par NEW ENGLAND BIOLABS). La réaction de ligation est effectuée pendant 48 heures à + 4°C (ou pendant 16 heures à + 15°C) dans une solution 60 mM Tris-HCl pH 7,5, 1 mM EDTA, 10 mM MgCl₂, 10 mM DTT, 1 mM ATP, en présence d'une unité de T4DNA ligase (BOEHRINGER) par µg de DNA, dans un rapport molaire de 100 (linker/ADN à bouts francs). Le mélange de ligation est utilisé pour transformer les bactéries E. coli HB101 (BOYER, ROULLAND-DUSSOIS, 1969) traitées au chlorure de calcium (protocole MANIATIS et al. 1982). Les bactéries sont ensuite étalées sur boîtes d'agarose LB contenant de l'ampicilline (100 µg/ml). Les plasmides sont extraits selon la technique décrite par BIRNBOIM et DOLY (1979) et triés dans le but d'isoler ceux contenant le site de restriction XhoI.

Un plasmide pAM1 (fig. 16) possédant le site de restriction XhoI à la place du site HindIII est retenu. Ce plasmide est préparé en grande quantité après lyse des bactéries au lysozyme-sodium dodécyl sulfate (GODSON et VAPNEK, 1973), et purification en gradient de chlorure de césium. L'ADN plasmidique prélevé sur le gradient est utilisable après extractions successives volume à volume par le butanol 1 (3 fois), le mélange 1 : 1 phénol saturé en TE (10 mM Tris-HCl pH 7,5, 1 mM EDTA) chloroforme, alcool isoamylique (24 : 1). La phase aqueuse extraite est précipitée par addition de 2 volumes d'éthanol 95 %, puis centrifugée et le culot repris dans un tampon TE.

### 2° Remplacement du site EcoRV (position 185) par un site BglII dans le plasmide modifié obtenu

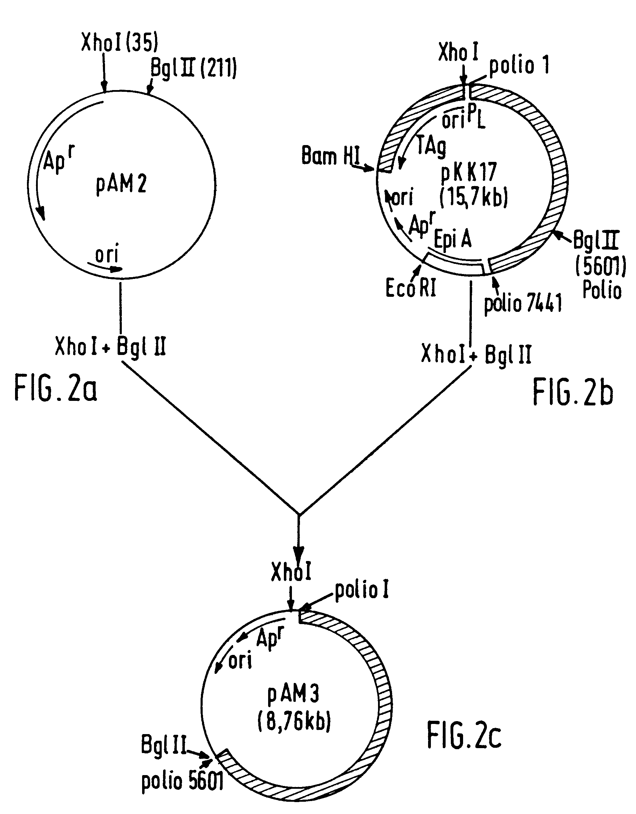

D'une façon semblable, le plasmide pAM1 est linéarisé au site EcoRV, déphosphorylé et mis en ligation avec un "linker" BglII phosphorylé (8 nucléotides). Des clones ampicilline résistants sont obtenus après transformation des bactéries HB101 par le mélange de ligation. Les plasmides sont préparés selon la technique précédemment décrite et analysés pour la présence du site de restriction BglII. Un plasmide pAM2 (fig. 1c ou fig. 2a) possédant les sites de restriction XhoI et BglII à la place des sites HindIII et EcoRV respectivement est purifié en gradient de chlorure de césium.

### II SOUS CLONAGE DU FRAGMENT XhoI-BglII DU cDNA DE POLIOVIRUS TYPE 1 MAHONEY DANS LE VECTEUR pAM2

Le sous-clonage d'un fragment comprenant les 5 601 premiers nucléotides de l'ADN complémentaire (cDNA) du poliovirus type 1 MAHONEY (sur 7 441 nucléotides) est réalisé de façon à ce que les sites de restriction à générer dans ce fragment par mutagénèse dirigée soient uniques dans la construction. Les nouveaux sites de restriction seront ainsi accessibles par des digestions totales par les enzymes de restriction correspondantes, afin que le fragment compris entre ces sites puisse être facilement délété et remplacé par une séquence étrangère de substitution.

Le "fragment polio" et le vecteur nécessaire à ce sous-clonage sont obtenus ainsi qu'il est décrit ci-après.

### 1° Fragment polio

Le fragment de restriction XhoI-BglII contenant les 5 601 premiers nucléotides du cDNA de polio (5638 paires de bases) est issu du plasmide pKK17 (K. KEAN et al. 1986)(fig. 2b) Ce plasmide contient la totalité du cDNA (représentée par une région hachurée) du poliovirus cloné immédiatement après le promoteur tardif de SV40 PL contenu dans le fragment (TAg) issu de SV40, ainsi que les éléments nécessaires à sa réplication autonome dans des cellules de singe (origine de réplication de SV40), promoteur de la transcription, séquence enhancer de la transcription, antigène T fonctionnel). Il contient également l'origine de réplication bactérienne et le gène codant pour la résistance à l'ampicilline.

Le plasmide pKK17 est digéré de façon totale par les enzymes de restriction XhoI et BglII (BOEHRINGER) auxquelles correspondent des sites uniques dans ce plasmide. Le fragment de 5 638 paires de base (pb) est isolé à partir d'un gel d'agarose à bas point de fusion (low melting) (WIESLANDER, 1979) purifié par deux extractions au phénol, une extraction par le mélange phénol/chloroforme : alcool isoamylique (IAA) et deux extractions par le mélange chloroforme : alcool isoamylique (IAA).

### 2° Vecteur

Parallèlement, le plasmide pAM2 est digéré de façon totale (sites uniques) par les enzymes de restriction XhoI et BglII. Le fragment BglII-XhoI (3 109 pb) contenant l'origine de réplication bactérienne et le gène de résistance à l'ampicilline est de même isolé et purifié à partir d'un gel d'agarose low melting.

### 3° Sous-clonage

Une réaction de ligation des deux fragments (rapport molaire insert/vecteur (2 : 1)) est effectuée. Les bactéries HB101 sont transformées avec le mélange de ligation. Des clones ampicilline-résistants sont obtenus. L'ADN plasmidique est extrait puis analysé sur la base de la taille des fragments générés après digestion par les enzymes XhoI et BglII.

On recueille pAM3 (fig. 2c ou fig 3a) qui possède la séquence des 5 601 premières paires de bases du cDNA de polio et on le purifie sur gradient de chlorure de césium.

### III CREATION DE NOUVEAUX SITES DE RESTRICTION DANS LA SEQUENCE 1-5 601 DU POLIOVIRUS

Les nouveaux sites de restriction sont créés par la technique de mutagénèse dirigée par oligonucléotide synthétique (MORINAGA et al. 1984), appliquée sur plasmide double brin, en l'occurence pAM3. A cette fin, il a été choisi de créer des sites Hpal ou EcoRV en position 2 756, et un side HindIII en position 2 786 dans la séquence du poliovirus. Ces deux positions encadrent exactement la boucle 93-103 de la protéine de capside VP1 qui correspond à un site antigénique neutralisant de type I du poliovirus, située entre deux feuillets Bêta. Les sites de restriction qui seront ainsi générés permettront de déléter la séquence nucléotidique codant pour les acides aminés de cette boucle et de la remplacer par une séquence hétérologue choisie en fonction de son rôle antigénique, avec pour objectif d'exposer cette séquence hétérologue à la surface du virion.

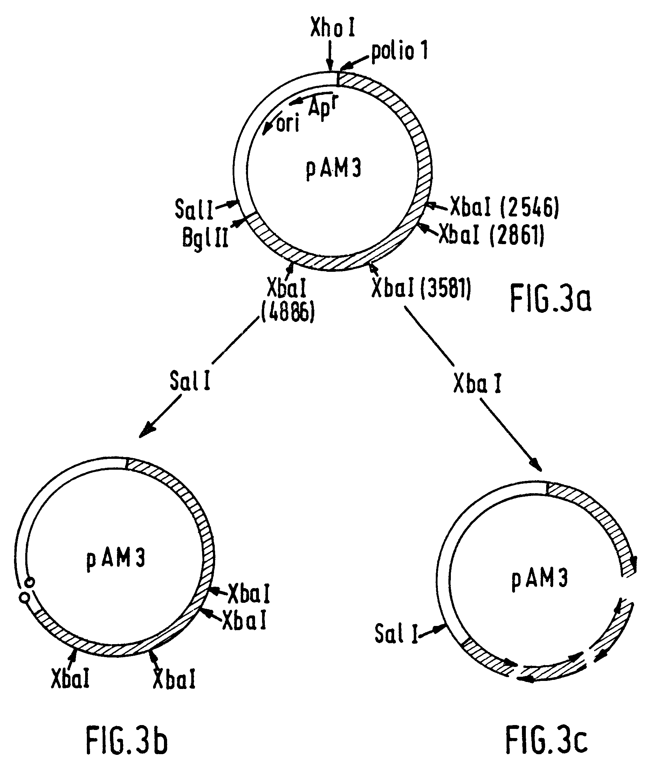

La mutation d'un seul nucléotide dans de l'ADN du poliovirus peut être suffisante pour engendrer un nouveau site de restriction. La technique est ici utilisée pour générer (grâce aux paires d'oligonucléotides synthétiques correspondants (cf. fig.3) des doubles mutants du poliovirus contenant, pour les premiers, des sites, HpaI et HindIII par réalisation de mutations ponctuelles dans les positions 2 757-2 791 respectivement et, pour les seconds, des sites EcoRV et HindIII par des mutations dans les positions 2 760 et 2 791 respectivement.

Ces techniques sont mises en oeuvre en utilisant, en sus des plasmides sus-indiqués, des oligonucléotides synthétiques. Ceux-ci comportent chacun une mutation ponctuelle par rapport à la séquence nucléotidique d'une partie du "fragment polio" ci-dessus. Leurs longueurs ont été choisies de façon à encadrer le nucléotide muté et à présenter un minimum d'homologie avec les autres séquences du poliovirus sauvage de MAHONEY. On procède notamment dans les conditions qui suivent.
1° Le plasmide pAM3 est digéré par Sal1 (en position 651 de la partie pBR327) pour générer un fragment linéaire I (fig. 3b).
2° Le plasmide pAM3 est digéré par XbaI, site présent aux positions 2 546, 2 861, 3 581, 4 886 du fragment de cDNA du poliovirus. On génère donc 4 fragments dont l'un (2 546-2 861) est éliminé après séparation sur gel d'agarose "low melting"(à bas point de fusion). On remarquera que le fragment éliminé contenait la séquence d'acide nucléique codant pour la susdite boucle 93-103 de la protéine de capside VP1 ainsi que les nucléotides qui l'entouraient dans ce qui sera appelé plus loin la "séquence poliosauvage".
   Au contraire les 3 autres fragments sont extraits. Ceux-ci présentent des tailles respectivement de 720 pb : (fragment IIa), 1 305 pb : (fragment IIb), et 6 405 pb : (fragment IIc). Ceci permettra ensuite d'obtenir après dénaturation et hybridation avec le fragment pAM3 digéré par Sal1 une région simple brin entre les positions 2 546-2 861, où l'on désire introduire les mutations.
3° Les fragments I, IIa, IIb, IIc sont individuellement purifiés par extraction au phénol, puis précipitation à l'éthanol 95 %. Ils sont dissous dans le tampon TE 10 mM Tris-HCl pH 7,5, 1 mM EDTA. Les fragments IIa, IIb, IIc sont ensuite réunis dans une même solution.
4° Les oligonucléotides ont été synthétisés dans un synthétiseur automatique, purifiés par chromatographie en phase liquide de haute performance (HPLC), puis par électrophorèse sur gel 20 % acrylamide contenant de l'urée 7M. Enfin, ils ont été phosphorylés par traitement à la T4 polynucléotide kinase (2 x 1unité /µg DNA) dans un tampon 50 mM Tris-HCl pH 9,0, 10 mM MgCl₂, 5 mM DTT, en présence d'ATP 1 mM pendant 2 x 1 heure à 37°C.

Ces oligonucléotides correspondent à des parties de la "séquence poliosauvage" dont la structure suit. En particulier, ils comportent une mutation d'un nucléotide du codon codant pour les acides aminés :
- 93 pour l'oligonucléotide A,
- 104 pour l'oligonucléotide B, et
- 94 pour l'oligonucléotide C.

Les nombres qui précèdent corresondent aux numérotations des acides aminés de la protéine VP1 codés par les codons correspondant de la "séquence polio sauvage" ci-après.

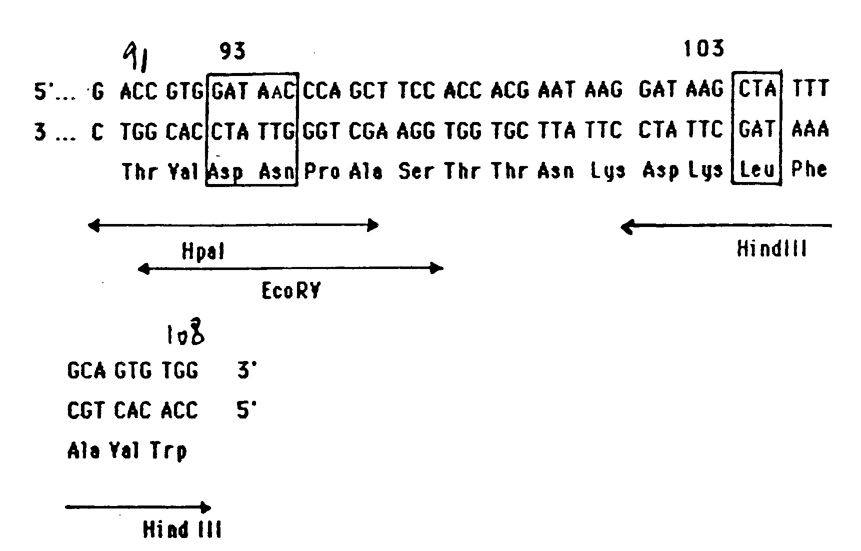
91 93 103

5'... G ACC GTG GAT AAC CCA GCT TCC ACC ACG AAT AAG GAT AAG CTA TTT

3 ... C TGG CAC CTA TTG GGT CGA AGG TGG TGC TTA TTC CTA TTC GAT AAA

Thr Yal Asp Asn Pro Ala Ser Thr Thr Asn Lys Asp Lys Leu Phe


Hpal HindIII


EcoRY

108

GCA GTG TGG 3'

CGT CAC ACC 5'

Ala Yal Trp


Hind III

On a fait apparaître sous les lignes identifiant les codons de la "séquence polio sauvage" et les acides aminés correspondants, l'indication des emplacements relatifs des oligonucléotides synthétiques (flèches) dont les formules suivent, grâce à une mutation ponctuelle dans leurs structures respectives (encadrés). Ces mutations ponctuelles sont illustrées par l'utilisation de lettres évidées, tant pour désigner le nucléotide de substitution résultant de cette mutation que pour identifier le changement de nature de l'acide aminé codé par le codon muté.

### Oligonucléotide A

HpaI 5' G ACC GTG GTT AAC CCA GC 3'
3' C TGG CAC CAA TTG GGT CG 5' Oligonucléotide synthétique
91 93 95 (18-mer)
Thr Val Val Asn Pro

Le site Hpal a été créé en modifiant le codon codant pour le résidu Aspartate (position 93 de VP1) en un codon codant pour la valine (A 2 757 > T).

### Oligonucléotide B

HindIII 5' G GAT AAG CTT TTT GCA GTG TGG 3'
3' C CTA TTC GAA AAA CGT CAC ACC 5' Oligonucléotide
102 104 108 (22-mer)
Asp Lys Leu Phe Ala Val Trp

Le site HindIII résulte d'une mutation muette (pour ce qui est de l'acide aminé exprimé) sur le résidu en position 104 VP1 (A 2 791 > T).

### Oligonucléotide C

EcoRY 5' CC GTG GAT ATC CCA GCT TCC 3'
3' GG CAC CTA TAG GGT CGA AGG 5' (20-mer)
92 94 97
Yal Asp Ile Pro Ala Ser

Le site EcoRV a été créé en modifiant le codon codant pour un résidu Asparagine (position 94 de VP1) en un codon codant pour une Isoleucine (A 2 760 > T).

Les fragments I et II (0,03 pmole de chaque) sont mélangés dans 10 µl d'eau, soit avec les oligonucléotides A et B, soit avec les oligonucléotides B et C, approximativement 20 pmole de chacun des oligonucléotides pour former dans les plasmides hybrides ultérieurs, soit les paires de sites Hpal + HindIII, soit les paires de sites EcoRV + HindIII. A ces mélanges sont ajoutés 2 µl de tampon polymérase-ligase 10 fois concentré (1 M NaCl, 65 mM Tris-HCl pH 7,5, 80 mM MgCl₂, 10 M B-mercaptoéthanol). Les fragments du mélange contenus dans un capillaire scellé sont dénaturés par incubation dans un bain marie d'eau bouillante pendant 3 minutes puis la renaturation des molécules se fait par refroidissement lent. Parmi l'ensemble des molécules formées, on trouve des molécules dont un brin de la matrice sauvage est hybridé soit avec les paires d'oligonucléotides A et B, soit avec les paires d'oligonucléotides B et C.

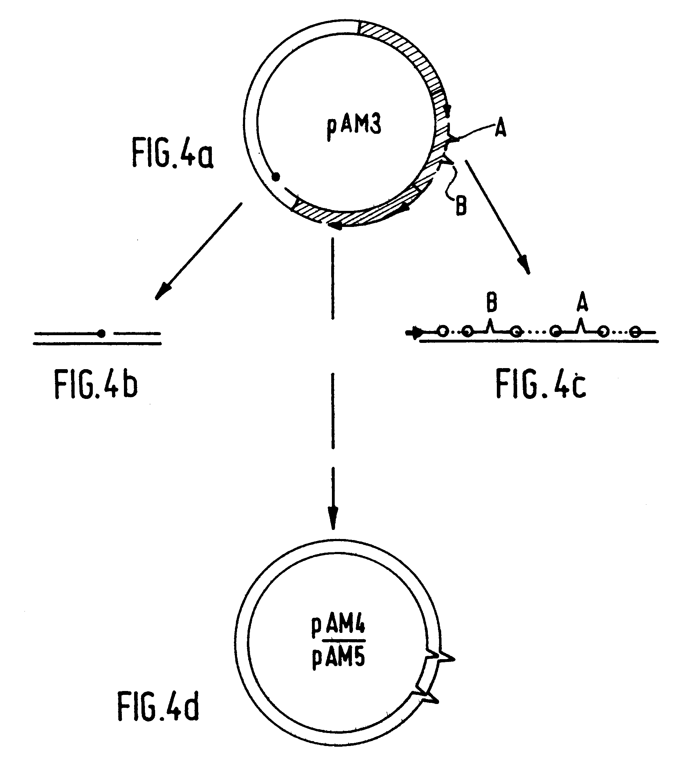

Le produit de ces hybridations est illustré dans la fig. 4a, en ce qui concerne plus particulièrement la première alternative ci-dessus. Il ne reste plus alors qu'à remplir les parties encore monobrins de la matrice illustrées dans les fig. 4b et 4c qui concernent des fragments comportant encore ces parties monobrins et dans lesquelles les insertions sont représentées par de petites saillies angulaires.

A cet effet, le mélange est traité par la DNA polymérase de KLENOV (3 unités + 4 dNTP + T4 DNA ligase (3 unités) pendant une nuit à 12,5°C afin que l'ADN simple brin soit converti en un ADN double brin, circulaire et contenant les mutations engendrées par les deux oligonucléotides.

Les bactéries HB101 sont transformées par le mélange ; sur plus de 1 000 transformants ampicilline résistants obtenus pour chaque construction, 600 transformants sont triés (screening) par hybridation de colonies (selon GRUNSTEIN et HOGNESS 1975). Les oligonucléotides synthétiques marquées au ³²p par la polynucléotide kinase servent ensuite de sonde d'hybridation pour identifier les bactéries transformées par les plasmides mutés (hybridations réalisées à 42°C, lavages réalisés à 53°C). Une dizaine de clones positifs sont retenus et utilisés pour retransformer des bactéries HB101 afin de séparer les plasmides mutants des "plasmides sauvages" pouvant être contenus dans le même transformant. Les plasmides préparés selon la méthode de BIRNBOIM et DOLY sont testés pour la présence des sites de restriction correspondants, soit Hpal et HindIII, soit EcoRV et HindIII.

Les plasmides retenus (fig. 4d) sont :
- pAM4 : sites Hpal et HindIII
- pAM5 : sites EcoRV et HindIII.

Les plasmides sont préparés et purifiés en CsCl puis séquencés pour confirmer les mutations. Le séquençage se fait selon la méthode de ZAGURSKY directement sur le plasmide préparé selon la méthode de BIRNBOIM et DOLY ou en chlorure de césium.

### IV. CONSTRUCTION DE PLASMIDES HYBRIDES

Les sites de restriction Hpal et HindIII uniques dans le plasmide pAM4 (ou EcoRV et HindIII de pAM5) permettent de déléter la séquence nucléotidique correspondant aux acides aminés 94 à 102 de VP1 du poliovirus, c'est à dire à l'épitope C3 et de la remplacer par n'importe quelle autre séquence codant pour un motif antigénique hétérologue, par exemple d'un entérovirus (poliovirus type 2 ou type 3 ou de l'hépatite A) ou de tout autre épitope hétérologue immunogène.

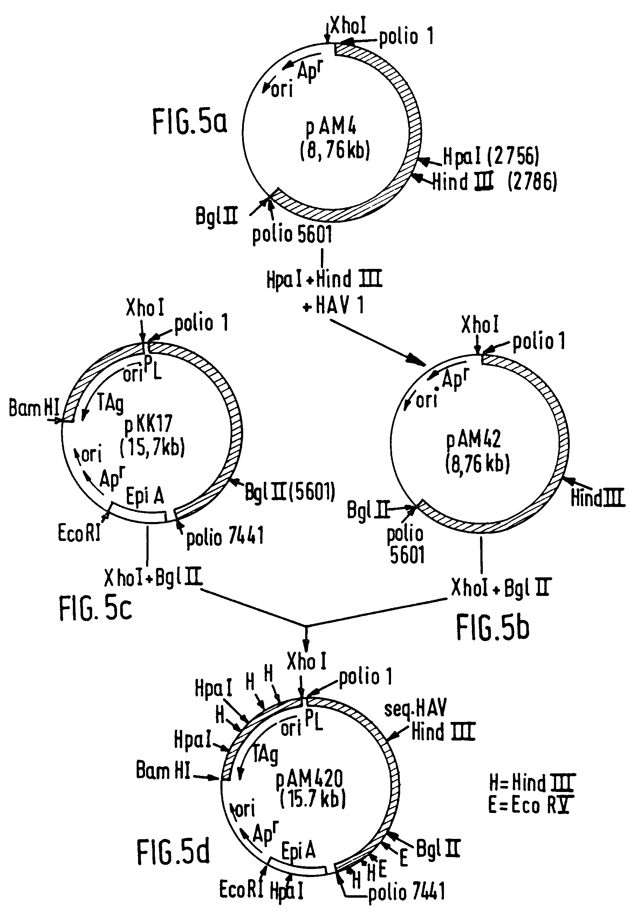

### Exemple de construction d'un plasmide poliovirus (hépatite A virus HAV) à partir de pAM4 (fig. 5a-5d).

Le plasmide pAM4 (également représenté à la fig. 4b) est digéré par les enzymes de restriction Hpal et HindIII, Hpal générant des bouts francs et HindIII des bouts cohésifs, puis déphosphorylé. La séquence d'hépatite A est introduite sous forme de deux oligonucléotides complémentaires synthétisés, purifiés et phosphorylés comme décrit ci-dessus, l'un d'entre eux étant complété par un A et l'autre précédé par une séquence AGCTT (voir ci-dessous) pour restaurer un site HindIII permettant la ligation à bouts cohésifs avec le vecteur. Les deux oligonucléotides encadrés hybridés entre eux comme montré ci-dessous, sont ensuite incorporés dans la séquence du vecteur poliovirus (dont les nucléotides et aminoacides extérieurs à la séquence HAV sont représentés par des lettres évidées), notamment en procédant comme indiqué ci-après. 
HindIII
93 72 YP1 HAV 81 103 104
5' GTG GTT AAG CCT GGA GAG TCT AGA CAT ACA TCA GAT AAG CTT TTT CGA 3'
3' CAC CAA TTC GGA CCT CTC AGA TCT GTA TGT AGT CTA TTC GAA AAA GCT 5'
Val Val Lys Pro Gly Glu Ser Arg His Thr Ser Asp Lys Leu Phe Ala
La réaction de ligation est effectuée, en présence de T4 DNA ligase pendant 48 h à + 4°C entre le vecteur délété et les 2 oligonucléotides hybridés dans un rapport molaire de 1/20. Les bactéries HB 101 sont transformées avec le mélange et les clones transformants sont analysés par hybridation de colonies avec l'un des 2 oligonucléotides de HAV utilisé comme sonde marquée au ³²p (hybridation à 37°C, lavages à des températures successives de 42°C à 68°C). Les plasmides sont extraits des clones positifs selon la méthode de Birnboim et Doly et sont testés pour la présence du site HindIII par digestion avec l'enzyme correspondante. Un plasmide pAM42 (fig. 5b) est retenu. Sa séquence dans la zone de l'insertion est contrôlée par séquençage selon la méthode décrite.

La séquence 1 à 5601 de poliovirus contenant une séquence hétérologue est ensuite replacée dans le plasmide pKK17 (représenté à nouveau dans la fig. 5c), de façon à reconstituer un cDNA complet de poliovirus. Pour cela, le plasmide pAM42 est digéré par les enzymes de restriction Xhol et BgIII et le fragment Xhol.BgIII (5641pb) contenant la séquence polio-HAV est isolé et purifié à partir d'un gel d'agarose "low-melting". Parallèlement, le plasmide pKK17 est également digéré par Xhol et BgIII et le fragment BgIII-Xhol (10028pb) contenant les 1840 dernières paires de bases de polio ainsi que les séquences nécessaires à la réplication du plasmide dans les bactéries et dans les cellules de primates, est isolé et purifié de la même façon.

Une réaction de ligation est effectuée entre les 2 fragments et les bactéries HB101 sont transformées par le recombinant. Les plasmides extraits des clones transformants sont analysés par doubles digestions par les enzymes de restriction Xhol et Hpal et/ou Xhol + HindIII et triés sur la base de la taille des fragments obtenus.

Un plasmide pAM420 (fig. 5d) est retenu : il contient la totalité du cDNA du poliovirus avec l'insérat de HAV, dans un plasmide infectieux pour les cellules de primates. Il a ensuite donné lieu à la production d'un poliovirus hybride, ci-après désigné v420, dans les conditions explicitées plus loin.

Le plasmide est séquencé selon la technique décrite afin de confirmer les séquences de l'insertion.

De la même façon, d'autres séquences peuvent être substituées au site antigénique I du poliovirus type 1 Mahoney, par exemple :
- la séquence 94 à 102 VP1 du poliovirus type 2 Lansing, grâce aux oligonucléotides synthétiques suivants : 
HindIII
93 94 VP1 T2 102 103 104
5' CTG GTT AAT GAT GCT CCA ACA AAG CGT GCC AGT AAG CTT TTT GCA 3'
3' CAC CAA TTA CTA CGA CGT TGT TTC GCA CGG TCA TTC GA A AAA CGT 5'
Val Val Asn Asp Ala Pro Thr Lys Arg Ala Ser Lys Leu Phe Ala
polio avec séquence poliovirus type2 Lansing polio
l'acide aminé
93 muté
 ou la séquence 97 à 107 de VP1 du HAV : 
Hind III

93 97 YP1 HAY 107 103

5' GTC GTT ACT TTT ACC TTC AAT TCA AAT AAT AAA GAG TAC AAG CTT TTT 3'

3' CAC CAA TGA AAA TGG AAG TTA AGT TTA TTA TTT CTC ATG TTC GAA AAA 5'

Val Val Thr Phe Thr Phe Asn Ser Asn Asn Lys Glu Tyr Lys Leu Phe

polio avec Séquence HAY polio

l'acide aminé

93 muté
 pour former les séquences d'ADN hybrides (dans laquelle les oligonucléotides synthétiques hybridés sont encadrés) contenant lesdites séquences VP1 de HAV incorporées dans le cDNA du poliovirus.
   Les constructions faites dans le plasmide pAM5 permettent d'éliminer la mutation engendrée par le site de restriction EcoRV puisqu'elle est contenue dans la séquence délétée alors que, dans le plasmide pAM4, la mutation due au site Hpal située juste en amont de la séquence délétée, subsiste (Valine au lieu d'Aspartate). Une comparaison des deux systèmes est intéressante pour l'étude du rôle de l'acide aminé 93.
   Par exemple on a construit dans pAM5 le plasmide hybride poliovirus/HAV 72-81 (également dénommé v520), caractérisé par l'insertion hétérologue ci-dessous :

Hind III
93 72 YP1 HAY 81 103
5' GTG GAT AAG CCT GGA GAG TCT AGA CAT ACA TCA GAT AAG CTT TTT GCA 3'
3' CAC CTA TTC GGA CCT CTC AGA TCT GTA TGT AGT CTA TTC GAA AAA CGT 5'
Val Asp Lys Pro Gly Glu Ser Arg His Thr Ser Asp Lys Leu Phe Ala
polio sauvage Séquence HAY polio

### V. ETUDE DANS DES CELLULES ANIMALES

Les plasmides hybrides type pAM420 sont utilisés pour transfecter, en présence de DEAE Dextran (McCutchan and Pagano, 1965), selon la méthode de Sompayrac and Danna (1981) des cellules de rein de singe CV1 ou VER0, ou des cellules humaines (HeLa, Hep2, etc..) Les DNA sont préparés par la méthode de Birnboim et Doly ou en gradient de chlorure de césium.

10⁶ cellules CV1 ou VER0 sont étalées sur des boites de Petri de 60mm et incubées avec 0,4ml d'un mélange à 0,25 µg/ml d'ADN dans le milieu de Eagle modifié par Dulbecco (DMEM) sans sérum, contenant 50 mM Tris-HCl, pH 7.5 et 500 µg/ml DEAE dextran. L'incubation se fait à 37°C dans une étuve à CO₂ pendant 2 heures. Les cellules sont ensuite lavées avec du DMEM sans sérum, puis entretenues dans du DMEM supplémenté par 5% de sérum de veau foetal. Après une dizaine de jours, lorsque l'effet cytopathique (ECP) est total, les cellules sont alors cassées par 5 congélations/décongélations successives dans la carboglace, centrifugées pendant 30mn à 1500 rpm pour éliminer les débris cellulaires ; le surnageant est utilisé comme mini-stock viral.

Les mini stocks viraux sont titrés sur plaque à 6 cupules : les cupules sont ensemencées avec 4.10⁵ cellules CV1 ou VER0 chacune, puis infectées par 0,2 ml des dilutions successives de la suspension virale dans le milieu DMEM sans sérum supplémenté avec 10 mM MgCl2. L'incubation se fait à 37°C dans une étude à CO₂ pendant 30 min. La suspension virale est alors retirée et les cellules recouvertes par une gélose contenant du DMEM avec 2% de sérum de veau foetal. Deux jours après l'infection, les plaques sont colorées au cristal violet : la gélose est retirée et la nappe cellulaire, perçée de plages de lyse, est colorée par une solution aqueuse contenant 10% formol, 20% éthanol et 2g/l de cristal violet. Les plages de lyse sont comptées dans la dilution adéquate. Le titre de la suspension virale est exprimée en unités formant plage (pfu)/ml.

Les mini-stocks sont utilisés pour fabriquer des maxi stocks de virus : pour cela, 5.10⁶ cellules CV1 ou 8.10.⁶ cellules HEp2 (cellules de cancer épithélial humain, sur lesquelles le poliovirus se développe mieux) sont étalées sur boites de Petri de 100 mm et infectées par la suspension virale à la multiplicité de 1 pfu/cellule. L'incubation se fait à 37°C dans une étuve à CO₂ pendant 1 heure. Les cellules sont ensuite entretenues dans du DMEM contenant 2% de sérum de veau foetal. 22 heures plus tard, quand l'ECP est total, les cellules sont traitées comme décrit pour l'obtention du mini-stock. Le maxi-stock est également titré.

La séquence de l'ARN viral extrait des virions composant ce maxi stock est contrôlées au niveau de l'insertion. Dès lors, à partir de ce maxi-stock, différentes analyses sont réalisées :
- caractérisation des virus : réactions avec des anticorps polyclonaux et/ou monoclonaux dirigés contre le poliovirus vecteur, et contre l'antigène auquel on a emprunté l'épitope hétérologue ; le cas échéant, on vérifie la perte de la capacité qu'avaient les anticorps monoclonaux spécifiques de la séquence remplacée dans le picornavirus mis en oeuvre de neutraliser celui-ci, par exemple perte de la capacité des anticorps monoclonaux C3 de neutraliser le poliovirus hybride ou chimère dérivé d'un poliovirus de type 1, du fait du remplacement de l'épitope C3 par l'épitope hétérologue ;
- injection des virus hybrides purifiés à des lapins, analyse des sérums obtenus (ELISA, immunoblot, seroneutralisation)
- marquage et préparation d'extraits de cellules infectées pour analyse des protéines de capside (notamment VP1) des virus hybrides en gel de polyacrylamide SDS(Laemmli,1970).
- au besoin injection à des lapins de peptides synthétiques correspondant aux séquences insérées dans le poliovirus : analyse des anticorps obtenus : reconnaissance et neutralisation des virus hybrides.

Chaque fois que l'on dispose de plusieurs épitopes d'insertion possible dans le picornavirus mis en oeuvre, on sélectionne dans un premier temps ceux qui présentent les meilleures caractéristiques de résistance à la chaleur et les meilleurs rendements de production par rapport au picornavirus non modifié.

Les picornavirus hybrides retenus peuvent ensuite être testés pour leur immunogénicité et leur capacité à neutraliser les agents pathogènes auxquels les épitopes insérés dans le picornavirus hybride correspondent, notamment après injection à des animaux de laboratoire (souris, cobayes, lapins). On retient les virus hybrides ou chimères ayant autant que possible la stabilité et les rendements du poliovirus en culture et capables d'induire chez l'animal des anticorps neutralisants à titre élevé, non seulement contre le poliovirus mais aussi contre les cibles retenues (par exemple HAV, HIV, rotavirus). Ces virus sont testés ensuite pour leur capacité à induire dans les systèmes animaux appropriés (singes, chimpanzés, etc..) une protection contre une infection d'épreuve du virus considéré.

En particulier pour des poliovirus modifiés par une séquence issue du virus de l'hépatite A (HAV), on peut tester l'immunogénicité du nouveau principe vaccinant comme suit :

Environ 10⁸ pfu de chaque stock viral clarifié sont mélangés volume à volume avec de l'adjuvant de Freund complet (AFC) et injectés à chaque lapin d'un lot de 4 lapins (femelles - race NewZeland blanche) par voie intradermique (ID) multipoints, sur le dos de l'animal. Un premier rappel est effectué trois semaines après l'immunisation, dans les mêmes conditions (10⁸ pfu de virus injectés par voie ID, en présence d'AFC). Les sérums issus de la saignée collectée deux semaines après le rappel sont analysés en séroneutralisation : on recherche la présence d'anticorps neutralisant HAV et, également, d'anticorps neutralisant PV-1.

Dans la méthodologie décrite ci-dessus pour la production de poliovirus hybrides ou chimères, on a directement utilisé le cADN des hybrides, cloné dans le plasmide infectieux pKK17 (décrit partie II-1), pour donner naissance aux virus correspondants, par transfection de cellules de primate.

Il est également possible de passer par l'intermédiaire d'une transcription in vitro des cADN et de générer les virus correspondants après transfection des cellules par les ARN de polarité positive obtenus in vitro. L'infectivité de ces ARN est supérieure à celle des clones de cDNA, lors des transfections dans les cellules de primates.

Pour cette seconde méthodologie, des cADN hybrides sont clonés, par exemple, derrière le promoteur du phage T7 (dans un plasmide décrit par S.van der Werf et al., 1986) ou le promoteur du phage SP6 de Salmonella (dans un plasmide décrit par G.Kaplan et al., 1985). Les cADNs sont transcrits dans un système acellulaire, utilisant la RNA polymérase de T7 purifiée ou la RNA polymérase de SP6 et susceptible de générer in vitro des copies d'ARN viral infectieux, de polarité positive. La très haute spécificité de l'ARN polymérase de T7 pour ses propres promoteurs, sa capacité à fabriquer des transcrits complets de longs cADN permettent de produire de grandes quantités d'ARN dans ce système. Ces ARN servent de matrice pour la traduction dans les cellules transfectées.

Le plasmide pT7PV1-5 décrit par S.van der Werf (1986) permet, de par sa construction, d'obtenir des ARN (+) contenant seulement 2 résidus G excédentaires en 5′ et 3 résidus excédentaires en aval du polyA en 3′. L'élimination des longues séquences excédentaires en 5′ et 3′ des ARN produits à partir des constructions initiales a permis d'augmenter l'infectivité des ARN de 10³ pfu/µg à 10⁵ pfu/µg (soit environ 5% de l'infectivité des ARN viraux extraits des virions).

En outre, ces méthodologies peuvent être appliquées d'une manière générale à des cADN hybrides sur une plus grande partie de leur génome. Par le même système de "cassette" encadrée par des sites de restriction que l'on peut générer par mutagénèse dirigée, on peut déléter la totalité de la partie P1 du génome du poliovirus et la remplacer par une région P1 d'un autre Picornavirus, et générer des virus hybrides (avec éventuellement complémentation par un système pouvant apporter des capsides de poliovirus exogènes si nécessaire).
On présente encore, ci-après et à titre d'exemples non limitatifs un certain nombre de peptides immunogènes neutralisants susceptibles d'être incorporés comme précédemment décrit dans les protéines de capside d'un poliovirus.

### EXEMPLES DE SEQUENCES IMMUNOGENES AYANT ETE INSEREES OU POUVANT ETRE INSEREES DANS UN VECTEUR CASSETTE APPROPRIE, TEL QUE pAM4 ou pAM5

Pour certains des poliovirus hybrides viables ont effectivement été obtenus, on indique ci-après sa désignation sous la forme d'une lettre "v", suivie d'un nombre, ainsi que la désignation du plasmide mis en oeuvre dans la construction du cADN intermédiaire. Les séquences hybrides correspondantes ont été insérées en lieu et place de l'épitope C3 du poliovirus.
1/ Séquences caractéristiques du virus de l'hépatite virale A (HAV) codant pour des peptides immunogènes neutralisants (J.COHEN et al(1986) J.VIROL 61, 50-59) notamment les séquences correspondant à :
   - la région 102-110 VP1 HAV (v545 ; pAM5) 
102                                    110
5' Ser Asn Asn Lys Glu Tyr Thr Phe Pro 3'
5' TCA AAT AAT AAA GAG TAC ACA TTT CCA A 3' (28-mer)
3' AGT TTA TTA TTT CTC ATG TGT AAA GGT TTCGA 5'
(32-mer)
   - la région 102-115 VP1 HAV 
102                                    110
5' Ser Asn Asn Lys Glu Tyr Thr Phe Pro Ile Thr Leu Ser
115
 
Ser 3'
5' TCA AAT AAT AAA GAG TAC ACA TTT CCA ATA ACC TTG TCT
TCG A 3' (43-mer)
3' AGT TTA TTA TTT CTC ATG TGT AAA GGT TAT TGG AAC AGA
AGC TTCGA (47-mer)
   - la région 99-106 de VP1-HAV
      Thr Phe Asn Ser Asn Asn Lys Glu
      entourée par les résidus Asp(93)-Asn(94), d'une part, et Lys(103), d'autre part, du VP1 du poliovirus
   - la région 67-76 de VP3 HAV (v564 ; pAM5)
      Asn Ala Ser Asp Ser Val Gly Gln Glu Ile
      entourée par les résidus Asp(93) et Lys (103) de VP1 du poliovirus.
2/ Séquences caractéristiques du virus de l'hépatite B codant pour des peptides immunogènes neutralisants de ce virus, ces peptides correspondant à
   - la région 120-145 du cadre de lecture codant pour pre-S (région N-terminale de la protéine moyenne : 33 et 36kD). 
120                                     130
5' Met Gln Trp Asn Ser Thr Ala Phe His Gln Thr Leu
                                       140
Gln Asp Pro Arg Val Arg Gly Leu Tyr Leu Pro Ala
Gly Gly 3'
 réf. A.R.NEURATH, S.B.H.KENT, N.STRICK (1984). Science, 224, 392-394.
   - la région 12-32 pre-S 
12                                  20
5' Met Gly Thr Asn Leu Ser Val Pro Asn Pro Leu Gly
                                30      32
   Phe Phe Pro Asp His Gln Leu Asp Pro 3'
 réf. A.R.NEURATH, S.B.H.KENT, N.STRICK, P.TAYLOR, C.E.STEVENS, Nature 315, 154-156 (1985)
   - la région 139-147 S 
139 147
5' Cys Thr Lys Pro Thr Asp Gly Asn Cys 3'
 ref. BHATNAGAR et al.(1982), PNAS 79, 4400-4404. THANAVALA et al.(1986), J. Exp. Med;164, 227-236
3/ Séquences codant pour des peptides immunogènes du virus de l'Herpes Simplex type-1, notamment
   - région 8-23 de la glycoprotéine D mature (résidus 33 à 48 de la séquence prédite de gD1). 
    8       10
5' Ser Leu Lys Met Ala Asp Pro Asn Arg Phe Arg Gly
    20          23
   Lys Asp Leu Pro 3'
 réf. COHEN et al.(1984), J. Virol. 49, 102.108.
   - région 340-356 de la glycoprotéine D (résidus 365 à 381 de la séquence prédite). 
340                                    350
5' His Arg Arg Thr Arg Lys Ala Pro Lys Arg Ile Arg
                  356
Leu Pro His Ile Arg 3'
 ref. EISENBERG et al.(1985), J.Virol. 53, 634-644
   - ou région codée par la séquence de la glycoprotéine D HSV-1 :
      R.J.WATSON, J.H.WEIS, J.S.SALSTROM, L.W.ENQUIST.(1982), Science 218, 381-384.
4/ Séquences codant pour un antigène de surface immunodominant du circumsporozoïte de plasmodium falciparum, agent de la Malaria, notamment motif antigénique présent sur la protéine CS (protéine du circumsporozoïte -412 acides aminés et contenant 41 répétitions d'un tétrapeptide, en position centrale de la protéine). Le motif antigène utilisable est alors constitué par un certain nombre, par exemple 4 répétitions du tétrapeptide - Pro Asn Ala Asn -
   réf. V.ENEA et al. (1984)- Science 225, 628-630.
   J.B. DAME et al. (1985)- Vaccines 85 -Cold Spring Harbor Laboratory (7-11)
   ref. sequence protéine CS : J.B.DAME et al.(1984) Science 225, 593-599.
5/ Epitope de virus de l'influenza comportant les résidus 138-164 de l'hémagglutinine réf : M.SHAPIRA, M.JIBSON, G.MULLER, R.ARNON (1984) PNAS 81, 2461-2465.
6/ Epitopes caractéristiques de la glycoprotéine enveloppe de HIV I et HIV 2, par exemple des épitopes importants pour la séroneutralisation localisés dans la gp120, tels que définis respectivement par les groupes Bolognesi et Putney (épitope spécifique de type) et par le groupe de Ho et collaborateurs (épitope spécifique de groupe) ou par Matsuhito et al, J. Virol. 1988, 62, 2107-2114. ; ou encore des épitopes de neutralisation à spécificité large, tels qu'identifiés dans la gp41, laquelle porte de surcroît la séquence hydrophobe nécessaire à la fusion de l'enveloppe du virus avec la membrane cellulaire (Robin Weiss).
   En particulier, on peut faire appel à l'épitope de Bolognesi-Putney (Putney et al, Science, 1986, 234, 1392-1395) (classiquement décrit sous la forme d'une boucle constituée des acides aminés 301 à 336 de la gp120). 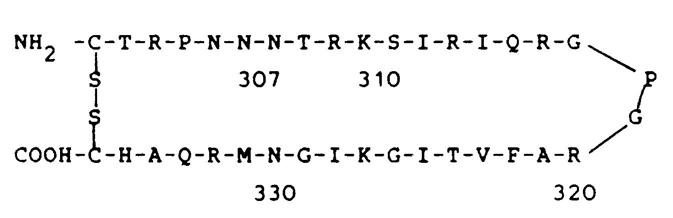 ou à des parties de la boucle correspondant aux séquences N T R K S I R I Q R G P G R (éventuellement suffisante pour induire des anticorps neutralisants spécifiques du variant type IIIB (LAV-1) ou à la séquence K G P G R V I, susceptible d'induire des anticorps neutralisant le variant RF.
   On peut aussi avoir recours au deuxième épitope de la gp120 (séquence des acides aminés 254 à 271 de Ho et al, 1988, Science 239, 1021) ;
   C T H G I R P V V S T Q L L L N G S
   qui induit des anticorps neutralisant (à des taux divers) tous les varians africains européens et américains testés ; ou à une partie de cet épitope : G I R P V V S T Q L. Ces épitopes sont particulièrement intéressants à inclure dans un vaccin puisqu'ils devraient permettre d'induire des anticorps à large spectre de neutralisation.
   A itre d'autres épitopes utilisables, on mentionne l'épitope de la gp41 correspondant aux acides aminés 735 à 752, à savoir : 
G I E E E G G E R D R D R S I R L V N G
740 750
 ou l'épitope (LASKY et al) qui paraît impliqué dans la reconnaissance du récepteur CD4 (acides aminés 404 à 425) : 
I K Q I I N M W Q K V G K A M Y A P P I S G
410 420
7/ Epitope de chaînes polypeptidiques VP3 et VP7 pour la protection contre le rotavirus ;
   tels que les épitopes identifiés récemment par Mackow, Harry Greenberg et al (Proc. Natl. Acad. Sci., 1988, 85, 645-649), en particulier :
   On mentionne en particulier des épitopes portés par VP3, les séquences des acides aminés 87 à 89 : 
L L A P T A A G V V V E G T
90
 148 à 150 : 
D V V K T T Q N G S Y S Q Y G P
150
 et 388 à 393 : 
G D Y S F A L P V G Q W P
390
 Ce dernier épitope paraît particulièrement intéressant car les anticorps neutralisants qui lui correspondent montrent une réactivité croisée importante avec plusieurs sérotypes de rotavirus.
   D'autres épitopes de neutralisation présents sur VP7 peuvent être utilisés, en particulier ceux coïncidant avec les régions des acides aminés 87 à 101 et 208 à 221 (Taniguchi, Channock et al, Virol., 1988, 62, 1870-1874), notamment ceux portant des séquences : 
T E A S T Q I N D G D W K D S L S Q et
G C Q T T N V D S F E M V A E N.
8/ Epitopes de la glycoprotéine du virus de la rage.
9/ Epitopes de l'antigène du choléra (brevet France n° 83.13828, publié sous le n° 2.551.088), etc..

Il va naturellement de soi que tout autre épitope pourra être mis en jeu pour la production de vaccins, de préférence des vaccins vivants, utilisant un poliovirus de virulence atténuée à titre de vecteur porteur (par exemple une souche SABIN). Ces vaccins seront destinés à l'homme ou à l'animal, selon la nature de l'épitope choisi et du vecteur choisi. Il apparaîtra aussi immédiatement à l'homme du métier que le picornavirus utilisé à titre de vecteur pourra être aussi, un virus virulent, auquel cas on se servira du recombinant obtenu pour fabriquer un vaccin inactive. Enfin, il revient au clinicien de déterminer les doses et le protocole d'administration du vaccin choisi, selon le mammifère auquel il sera destiné.

A toutes fins utiles on indique encore ci-après les références des articles des Auteurs auxquels il a été fait référence ci-dessus.

L'expression "épitope" telle qu'elle a été utilisée dans le cadre du présent texte n'est pas limitée à la seule séquence d'aminoacides auxquels est attribuée la capacité d'induction in vivo d'anticorps neutralisants contre l'antigène auquel cet épitope a été emprunté. L'expression doit être entendue comme englobant éventuellement des séquences d'acides aminés plus ou moins courtes qui, normalement, entourent l'épitope (au sens restreint de l'expression) dans l'antigène auquel il a été emprunté. à condition que tous les autres épitopes normalement exposés du poliovirus hybride soient reconnus par les anticorps spécifiques qui leur correspondent, et produits à partir du poliovirus natif, ou de protéines obtenues à partir de celui-ci.

On peut ainsi aussi procéder au transfert de substitutions réalisées dans le cDNA d'une souche sauvage (Mahoney) dans le cDNA d'une souche Sabin. L'invention permet donc la mise au point de vaccins chimères vivants utilisables par voie buccale. Cela est en particulier envisageable pour la production de vaccins contre l'hépatite A et les diarrhées à rotavirus. Le sous-clonage direct devenu possible d'un fragment du plasmide pAM5 substitué dans du cDNA Sabin devrait permettre de répondre à ce souci.

Il va d'ailleurs de soi que toutes les opérations qui ont été décrites dans ce qui précède s'appliquent de la même manière à un cADN dérivé d'une souche Sabin de type I elle-même, par exemple par la substitution dans ce cADN d'un oligonucléotide contenant la séquence codant pour l'épitope hétérologue choisi à la séquence endogène codant pour le peptide correspondant essentiellement à l'épitope C3 de la protéine VP1 de la souche Mahoney, en l'occurrence :

His Val Val Gln His
Arg Ser Arg Ser Glu Ser Ser Ile Glu Ser
Phe Phe Ala Arg Gly Ala Cys Val Ala Ile
Ile Thr Val Asp Asn Ser Ala Ser Lys Thr
Asn Lys Asp Lys Leu Phe Thr

### REFERENCES

Birnboim,H.C. and Doly,J. (1979) Nucl.Acids Res, 7, 1513-1522
Boyer,H.W. and Roulland-Dussois,D. (1969) J.Mol.Biol., 41, 459-472
Emini,E.A., Hughes,J.V., Perlow,D.S. and Boger,J. (1985) J.Virol., 55, 836-839
Godson,G.N. and VaepnekD. (1973) Biochim.Biophys.Acta, 299,516-520
Grunstein,M. and Hogness,D.S. (1975) Proc.Natl.Acad.Sci.USA, 72, 3961-3965
Hogle,J.M.,Chow,M. and Filman,D.J. (1985) Science, 229, 1358-1365
Kaplan,G., Lubinski,J., Dasgupta,A. and Racaniello,V.R. (1985) Proc.Natl.Acad.Sci.USA, 82, 8424-8428
Kean,K.M., Wychowski,C., Kopecka,H., and Girard,M. (1986) J.Virol., 59, 490-493
Kitamura,N., Semler,B.L., Rothberg,P.G., Larsen, G.R., Adler,C.J., Dorner,A.J.,
Emini,E.A., Hanecak,R., Lee,J.J. van der Werf,S. , Anderson,C.W. and Wimmer,E. (1981) Nature, 291, 547-553
Laemmli,U.K. (1970) Nature, 680-685
Maniatis,T., Fritsch,E.F., Sambrook,J. (1982) Molecular Cloning . A Laboratory Manual.Cold Spring Harbor Laboratory Press, N.Y
McCutchan,J.M. and Pagano,J.S. (1968) J.Natl.Cancer Inst., 41, 351-356
Morinaga,Y, Franceschini,T., Inouye,S and Inouye,M. (1984) Biotechnology,636-639
Racaniello,V.R and Baltimore,D. (1981) Proc.Natl.Acad.Sci.USA. 78, 4887-4891
Rossmann,M.A., Arnold,E., Erickson,J.W., Frankenberger,E.A., Griffith,J.P., Hecht,H.J., Johnson,J.E., Kamer, G., Luo,M., Mosser, A.G., Rueckert,R.R., Sherry,B. and Vriend,G. (1985) Nature, 317,145-153
Semler,B.L., Dorner,A.J., and Wimmer,E (1984) Nucl.Acids Res., 12, 5123-5141
Soberon,X., Covarrubias,L. and Bolivar,F. (1980) Gene,9,287-305
Sompayrac,L.M. and Danna,K. (1981) Proc.Natl.Acad.Sci;USA,78,7575-7578
van der Werf,S., Bregégère,F., Kopecka,H., Kitamura,N., Rothberg,P.G., Kourilsky,P., Wimmer,E. and Girard,M. (1981) Proc.Natl.Acad.Sci.USA 78, 5983-5987
van der Werf,S., Bradley,J. Wimmer,E., Studier,F.W., and Dunn,J.J. (1986) Proc.Natl.Acad.Sci.USA, 83, 2330-2334
Wieslander,L. (1979) Anal.Biochem, 98, 305-309
Wychowski,C., van der Werf,S., Siffert,O., Crainic,R., Bruneau,P., and Girard,M. (1983) The EMBO J., 2,2019-2024
Zagursky,R.J., Baumeister,K., Lomax,N., and Berman,M.L. (1985) Gene Analysis Techniques, 2, 89-94

## Revendications

1. Poliovirus hybride viable contenant, en lieu et place de l'épitope situé entre les acides aminés 93 et 104 de la protéine VP1 ou d'une partie de cet épitope, un épitope caractéristique d'une protéine vaccinante hétérologue vis-à-vis des protéines de ce poliovirus, tous les autres épitopes normalement exposés du poliovirus étant reconnus par les anticorps spécifiques qui leur correspondent et produits à partir du poliovirus natif ou de protéines obtenues à partir de celui-ci.

2. Poliovirus selon la revendication 1 caractérisé en ce que l'épitope hétérologue jouxte directement des séquences d'acides aminés appartenant à la protéine VP1 en l'absence de résidus amino-acides ou de peptides de liaison.

3. Poliovirus selon l'une quelconque des revendications précédentes caractérisées en ce que l'épitope hétérologue est l'épitope :
Thr-Phe-Asn-Ser-Asn-Asn-Lys-Glu-
de la protéine VP1 du virus de l'hépatite A, en lieu et place des acides aminés 95 à 102 de la protéine VP1 du poliovirus.

4. Poliovirus selon la revendication 1 caractérisé en ce que l'épitope hétérologue est l'épitope :
Asn-Ala-Ser-Asp-Ser-Val-Gly-Gln-Gln-Ile
de la protéine VP3 du virus de l'hépatite A, en lieu et place des acides aminés 94 à 102 de la protéine VP1 du poliovirus.

5. Poliovirus selon la revendication 1 caractérisé en ce que l'épitope hétérologue comprend au moins une partie de la séquence : 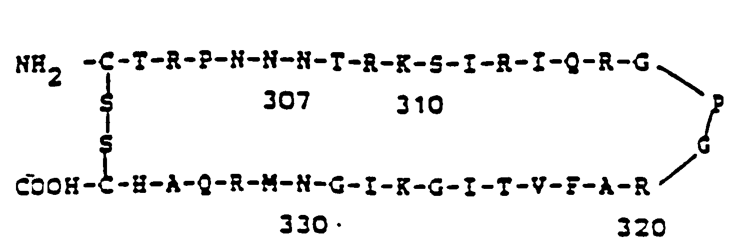
de la protéine GP120 de HIV.

6. Poliovirus selon l'une quelconque des revendications précédentes caractérisé en ce que l'épitope hétérologue est l'épitope :
K G P G R V I
de la protéine GP120 de HIV isolat RF1.

7. Poliovirus selon la revendication 1 caractérisé en ce qu'il est constitué d'un poliovirus humain Sabin-type 1 et en ce qu'il contient, en lieu et place de l'épitope situé entre les acides aminés 93 et 104 de la protéine VP1 ou d'une partie de cet épitope, les séquences équivalentes des souches Sabin-type 2 ou type 3.

8. Poliovirus selon la revendication 1 caractérisé en ce qu'au moins l'un des deux acides aminés jouxtant la jonction entre la séquence de la protéine VP1 native et l'épitope hétérologue est modifié suite à la création d'un site de restriction dans le cADN constituant la réplique de son génome.

9. L'ARN du poliovirus hybride selon l'une quelconque des revendications précédentes.

10. Le cADN résultant de la reverse-transcription de l'ARN de la revendication 9.

11. Procédé de fabrication d'un poliovirus hybride selon l'une quelconque des revendications 1 à 10, caractérisé en ce que, partant d'un cADN obtenu à partir de l'ARN viral :
- on introduit de part et d'autre de la séquence codant pour l'épitope situé entre les acides aminés 93 et 104 de la protéine VP1 d'un poliovirus ou pour une partie de cet épitope, des sites de restriction spécifiques dans le cADN ;
- on clive le cADN au niveau de ces sites spécifiques par les enzymes de restriction correspondantes ;
- on reconstitue un cADN recombinant hybride formé, d'une part, à partir de tous les éléments de l'ARN viral ou du cADN nécessaire à la réplication ultérieure du virus et, d'autre part, une séquence codant pour un épitope immunogène de substitution en lieu et place de la séquence codant pour l'épitope naturel ;
- on transfecte une culture cellulaire sensible avec cet ADN recombinant ;
- et l'on recueille enfin le poliovirus recombinant correspondant.

12. Vecteur-cassette comprenant une séquence d'ADN du poliovirus dont les nucléotides encadrant ceux codant pour les acides aminés 93 à 104, et de préférence 94 à 102, de la protéine VP1, sont modifiés pour créer des sites de restriction permettant de déléter la séquence nucléotidique correspondant auxdits acides aminés.

13. Vaccin actif contre un agent pathogène déterminé, dont le principe actif est constitué par un poliovirus hybride viable selon la revendication 1, ladite protéine vaccinante l'étant contre la maladie dûe au susdit agent pathogène détermine.

## Claims

1. Viable hybrid poliovirus containing an epitope characteristic of a vaccinating protein heterologous with respect to the proteins of this poliovirus in place of all or part of the epitope situated between the amino acids 93 and 104 of the VF1 protein, all of the other epitopes of the poliovirus normally exposed being recognized by the corresponding specific antibodies which are produced against the native poliovirus or against proteins obtained from the latter.

2. Poliovirus according to Claim 1, characterized in that the heterologous epitope is directly connected to amino acid sequences belonging to the VP1 protein in the absence of linking amino acid residues or peptides.

3. Poliovirus according to either of the preceding Claims, characterized in that the heterologous epitope is the epitope:
Thr-Phe-Asn-Ser-Asn-Asn-Lys-Glu-
of the VP1 protein of the hepatitis A virus in place of the amino acids 95 to 102 of the VP1 protein of the poliovirus.

4. Poliovirus according to Claim 1 characterized in that the heterologous epitope is the epitope:
Asn-Ala-Ser-Asp-Ser-Val-Gly-Gln-Gln-Ile
of the VP3 protein of the hepatitis A virus in place of the amino acids 94 to 102 of the VP1 protein of the poliovirus.

5. Poliovirus according to Claim 1, characterized in the heterologous epitope comprises at least a part of the sequence: 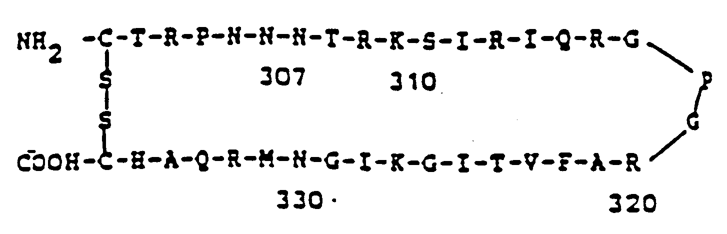 of the GP120 protein of HIV.

6. Poliovirus according to any one of the preceding Claims characterized in that the heterologous epitope is the epitope:
K G P G R V I
of the GP120 protein of the RF1 isolate of HIV.

7. Poliovirus according to Claim 1 characterized in that it is constituted by a Sabin type 1 human poliovirus and in that it contains in place of all or part of the epitope situated between the amino acids 93 and 104 of the VP1 protein the equivalent sequences of the Sabin strains type 2 or type 3.

8. Poliovirus according to Claim 1 characterized in that at least one of the two amino acids linked to the junction between the sequence of the native VP1 protein and the heterologous epitope is modified as a result of the creation of a restriction site in the cDNA constituting the replica of its genome.

9. The RNA of the hybrid poliovirus according to any one of the preceding Claims.

10. The cDNA resulting from the reverse transcription of the RNA of Claim 9.

11. Procedure for the production of a hybrid poliovirus according to any one of the Claims 1 to 10, characterized in that starting from a cDNA produced from the viral RNA:
- specific restriction sites are introduced into the cDNA on either side of the sequence coding for all or part of the epitope situated between the amino adds 93 and 104 of the VP1 protein of a poliovirus;
- the cDNA is cleaved at these specific sites by the corresponding restriction enzymes;
- a hybrid recombinant cDNA is reconstituted, formed, on the one hand, from all of the elements of the viral RNA or cDNA necessary for the subsequent replication of the virus and, on the other, a sequence coding for a substitute immunogenic epitope in place of the sequence coding for the natural epitope;
- a sensitive cell culture is transfected with this recombinant DNA;
- and finally the corresponding recombinant poliovirus is harvested.

12. Cassette vector comprising a DNA sequence of the poliovirus the nucleotides of which flanking those coding for the amino acids 93 to 104 and preferably 94 to 102 of the VP1 protein are modified to create restriction sites making it possible to delete the nucleotide sequence corresponding to said amino acids.

13. Vaccine active against a specific pathogenic agent the active ingredient of which is constituted by a viable hybrid poliovirus according to Claim 1, said protein vaccinating against the disease due to the above-mentioned specific pathogenic agent.

## Patentansprüche

1. Lebensfähiges Poliovirus-Hybrid, enthaltend anstelle des Epitops, das zwischen den Aminosäuren 93 und 104 des Proteins VP1 liegt, oder eines Teils des Epitops ein Epitop, das charakteristisch ist für ein gegenüber den Proteinen des Poliovirus heterologes Vakzinprotein, wobei alle anderen normalerweise exponierten Epitope des Poliovirus durch die ihnen entsprechenden spezifischen Antikörper erkannt und vom nativen Poliovirus oder von von diesem erhaltenen Proteinen produziert werden.

2. Poliovirus nach Anspruch 1, dadurch gekennzeichnet, daß das heterologe Epitop in Abweseheit von Aminosäureresten oder Verknüpfungspeptiden direkt neben den Aminosäuresequenzen liegt, die zum Protein VP1 gehören.

3. Poliovirus nach einem der vorangehenden Ansprüche, dadurch gekennzeichnet, daß das heterologe Epitop das folgende Epitop:
Thr-Phe-Asn-Ser-Asn-Asn-Lys-Glu-
des Proteins VP1 des Hepatitis A-Virus ist, anstelle der Aminosäuren 95 bis 102 des Proteins VP1 des Poliovirus.

4. Poliovirus nach Anspruch 1, dadurch gekennzeichnet, daß das heterologe Epitop das folgende Epitop:
Asn-Ala-Ser-Asp-Ser-Val-Gly-Gln-Gln-Ile
des Proteins VP3 des Hepatitis A-Virus ist, anstelle der Aminosäuren 94 bis 102 des Proteins VP1 des Poliovirus.

5. Poliovirus nach Anspruch 1, dadurch gekennzeichnet, daß das heterologe Epitop mindestens einen Teil der folgenden Sequenz: 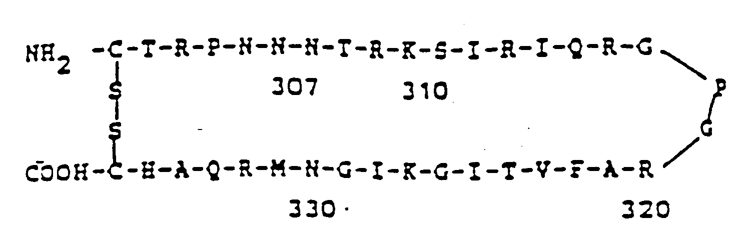 des Proteins GP120 von HIV umfaßt.

6. Poliovirus nach einem der vorangehenden Ansprüche, dadurch gekennzeichnet, daß das heterologe Epitop das folgende Epitop:
K G P G R V I
des Proteins GP 120 vom HIV-Isolat RF1 ist.

7. Poliovirus nach Anspruch 1, dadurch gekennzeichnet, daß es aus einem menschlichen Poliovirus vom Sabin-Typ 1 besteht und daß es anstelle des Epitops, das zwischen den Aminosäuren 93 bis 104 des Proteins VP1 liegt, oder eines Teils dieses Epitops die äquivalenten Sequenzen der Stämme vom Sabin-Typ 2 oder 3 enthält.

8. Poliovirus nach Anspruch 1, dadurch gekennzeichnet, daß mindestens eine der zwei Aminosäuren die an der Verbindung zwischen der Sequenz des nativen Proteins VP1 und dem heterologen Epitop liegen, durch die Erzeugung einer Restriktionsstelle in der cDNA geändert ist, die das Replikat seines Genoms darstellt.

9. RNA des Poliovirus-Hybrids nach einem der vorangehenden Ansprüche.

10. cDNA, erhältlich durch die reverse Transkription der RNA nach Anspruch 9.

11. Verfahren zur Herstellung eines Poliovirus-Hybrids nach einem der vorangehenden Ansprüche 1 bis 10, dadurch gekennzeichnet, daß man ausgehend von einer cDNA, die von der viralen RNA erhalten wird:
- auf beiden Seiten der Sequenz, die das Epitop, das zwischen den Aminosäuren 93 bis 104 des Proteins VP1 eines Poliovirus liegt, oder einen Teil dieses Epitops codiert,, spezifische Restriktionsstellen in die cDNA einführt,
- die cDNA an den spezifischen Stellen durch die entsprechenden Restriktionsenzyme spaltet,
- eine rekombinante hybride cDNA herstellt, die zu einem Teil aus allen Elementen der viralen RNA oder der für die spätere Replikation des Virus notwendigen cDNA und zum anderen Teil aus einer Sequenz besteht, die ein immunogenes Epitop codiert, als Ersatz für die Sequenz, die das natürliche Epitop codiert,
- eine sensitive Zellkultur mit dieser rekombinanten DNA transfiziert ist,
- und dann das entsprechende rekombinante Poliovirus gewinnt.

12. Vektorkassette, umfassend eine DNA-Sequenz des Poliovirus, dessen Nucleotide, die die des Proteins VP1 einrahmen, die die Aminosäuren 93 bis 104, vorzugsweise 94 bis 102, des Proteins VP1, so modifiziert werden, daß Restriktionsstellen erzeugt werden, die die Deletion der Nucleotidsequenz ermöglichen, die diesen Aminosäuren entspricht.

13. Impfstoff, der gegen ein bestimmtes pathogenes Mittel aktiv ist, dessen Wirkstoff aus einem lebensfähigen Poliovirus-Hybrid nach Anspruch 1 besteht, wobei das Vakzinprotein gegen die durch das bestimmte pathogene Mittel verursachte Krankheit wirkt.
